(19) **Europäisches Patentamt / European Patent Office / Office européen des brevets**

(11) **EP 4 321 865 A1**

(12) # EUROPEAN PATENT APPLICATION
published in accordance with Art. 153(4) EPC

(43) Date of publication:
**14.02.2024 Bulletin 2024/07**

(21) Application number: **22785021.1**

(22) Date of filing: **08.04.2022**

(51) International Patent Classification (IPC):
**G01N 33/574** (2006.01)　　**G01N 33/50** (2006.01)
**A61P 35/00** (2006.01)　　**A61K 39/00** (2006.01)

(52) Cooperative Patent Classification (CPC):
**A61P 35/00; G01N 33/57492; G01N 2800/52**

(86) International application number:
**PCT/KR2022/005128**

(87) International publication number:
**WO 2022/216112 (13.10.2022 Gazette 2022/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.04.2021　US 202163172950 P**
　　　　　　**12.04.2021　US 202163173550 P**

(71) Applicant: **Cytogen, Inc.**
**Seoul 05854 (KR)**

(72) Inventor: **JEON, Byung Hee**
**Seongnam-si Gyeonggi-do 13611 (KR)**

(74) Representative: **Mewburn Ellis LLP**
**Aurora Building**
**Counterslip**
**Bristol BS1 6BX (GB)**

(54) **METHOD FOR TREATING CANCER USING IMMUNE CHECKPOINT INHIBITOR**

(57)　Disclosed is a method of treating cancer using an immune checkpoint inhibitor. More specifically, disclosed is a method of treating cancer by checking expression of immune checkpoint protein and lymphoid cell- or myeloid cell-specific protein in circulating tumor cells isolated from blood, selecting, as a patient to whom a cancer immunotherapy drug is applicable, the patient found to be positive for expression of the immune checkpoint protein and negative for expression of the lymphoid cell- or myeloid cell-specific protein, and administering an immune checkpoint inhibitor to the patient. In the method, expression of immune checkpoint protein and expression of lymphoid cell- or myeloid cell-specific protein in a liquid biopsy are simultaneously checked, and then a patient is selected by eliminating false-positive circulating tumor cells. Thus, the method has high accuracy and high commercial utility, and thus is useful for cancer diagnosis and treatment.

FIG. 1

EP 4 321 865 A1

**Description**

Field of the Invention

The present invention relates to a method of treating cancer using an immune checkpoint inhibitor, and more particularly, to a method of treating cancer by checking expression of immune checkpoint protein and lymphoid cell- or myeloid cell-specific protein in circulating tumor cells isolated from blood, selecting, as a patient to whom a cancer immunotherapy drug is applicable, the patient found to be positive for expression of the immune checkpoint protein and negative for expression of the lymphoid cell- or myeloid cell-specific protein, and administering an immune checkpoint inhibitor to the patient.

Description of the Related Art

Cancer is one of the deadliest threats to human health. In the United States, cancer affects nearly 1.3 million new patients each year and is the second leading cause of death after heart disease, and one in four people die from cancer. In addition, cancer is expected to surpass cardiovascular disease as the number one cause of death within 5 years. Solid tumors account for the majority of these deaths.

Various methods for treating cancer have been tried, but the clinical results and prognosis of each patient did not always match the reported data, and thus the overall five-year survival rate of all cancer patients has only improved by about 100 over the past 20 years. Therefore, when the most appropriate treatment for cancer patients with heterogeneous characteristics is provided by predicting the responsiveness of the cancer patients to therapeutic drugs and the prognosis of the cancer patients, it is possible to avoid toxicity associated with unnecessary treatment and ultimately enhance therapeutic effects.

According to this necessity, in recent years, studies on companion diagnostics, which means approved diagnostics capable of providing information for the selection of an appropriate targeted anticancer drug and treatment method based on the systematic analysis results of the patient's individual factors, have been actively conducted. Companion diagnostics can provide a clear clinical rationale for prescription according to the doctor's diagnosis, and can provide appropriate treatment to the patient, thereby increasing cancer treatment efficiency as well as reducing the misuse of targeted anticancer drugs to contribute to the financial soundness of the national health insurance. Currently, the companion diagnostics market is growing in the fields of treatment of breast cancer, lung cancer, colorectal cancer, stomach cancer, melanoma, and the like, and in particular, the breast cancer and lung cancer treatment fields are expected to lead the market growth. As pharmaceutical companies have reduced the cost of developing new drugs and the demand for targeted therapies has increased, the global market for companion diagnostics has grown at a high rate every year.

Meanwhile, immunotherapy uses the patient's own immune system to attack cancer, irrespective of the origin. The immune system is regulated by a network of checks and balances that evolved to attack foreign invaders such as bacteria and viruses. However, cancer can evade the immune system by expressing proteins (e.g., PD-L1 and PD-L2) that suppress the immune system from attacking cancer cells.

In particular, the interaction between tumor cells and T cells involves contact between the major histocompatibility complex (MHC) on tumor cells and the T cell receptor (TCR) on T cells. Upon contact between the MHC and the T cell receptor, the T cells are activated and the tumor cells are destroyed.

If tumor cells express the immune checkpoint protein PD-L1 on their surface, they can evade T cell immunosurveillance. PD-L1, when present, binds to PD-1 expressed by T cells and suppresses T cell immunosurveillance by blocking T cell activation.

Immune checkpoint inhibitors that can block PD-L1/PD-1 interaction have been developed. Such drugs permit the T cell immunosurveillance mechanism to again function normally, and tumor cells can thus be destroyed through the normal immune response in the subject. Blockage of CTLA-4 on T cells can have a similar effect (Hodi et al., N. Engl. J. Med. Vol. 363, pp. 711-23, 2010).

The key to effective use of immune checkpoint inhibitors is determining whether a particular subject having cancer will respond to the drugs. If an antibody which binds to PD-L1 or PD-1 and that serves as an immune checkpoint inhibitor is administered to a patient whose tumor cells do not express PD-L1, the treatment will be ineffective. So far, the responsiveness of these immune checkpoint inhibitors is only 20 to 30% (Herbst RS, et al. The New England Journal of Medicine. Vol. 383(14), pp. 1328-39, 2020).

As a method of measuring the PD-L1 expression rate, calculating the tumor proportion score (TPS) of PD-L1 in the tissue cells of a patient is currently the recommended method. For example, in order for pembrolizumab (Keytruda) and nivolumab (Opdivo), which are antibodies that act specifically against PD-1, to work effectively, the expression rate of PD-L1 detected in the patient's biopsy should be a certain percentage (%) or more, and in order to be eligible for health insurance benefits, the expression rate of PD-L1 should be 100 or more for Opdivo and 50% or more for Keytruda.

For atezolizumab (Tecentriq), the expression rate of PD-L1 in both tumor cells and tumor-infiltrating immune cells should be 5% or more.

However, obtaining cancer cells via tumor biopsies for PD-L1 expression surveys has serious drawbacks that include pain and discomfort to patients, the inability to survey more than an isolated area of the tumor, and the potential for protein expression profiles to change over time in the tumor microenvironment. In addition, re-biopsy also causes invasiveness and other complicated problems. In addition, patient identification criteria differ between immune checkpoint inhibitors, and thus it is very difficult to select the most effective treatment for the patient.

To overcome these shortcomings, liquid biopsies containing circulating tumor cells (CTCs), circulating cell-free DNA (cfDNA) and exosomes have recently attracted attention as a new solution (Rijavec E, et al., Cancers. Vol. 12(1):17, 2020). Blood-based biopsies are an improvement over tissue biopsies in that they can provide real time sequential tracking of cells shed by the tumor or that otherwise break-off.

Blood samples can be more easily obtained and obtained more often from a patient. Furthermore, changes in protein expression profiles can be monitored over time. Circulating tumor cells (CTCs) are one cancer-associated cell type that can easily be isolated from the peripheral blood and that can be used as a substitute for tumor cells obtained from tissue biopsies. CTCs are tumor cells broken off from the solid tumors into the blood stream. CTCs can be found in blood of carcinomas, sarcomas, neuroblastomas and melanomas patients. The identification of additional cell types that can be obtained from blood-based biopsies will be critical to further development of the use of this technique for identifying cancer patients who will benefit from treatment with immune checkpoint inhibitors.

Under this technical background, the present inventors have made extensive efforts to develop a CTC-based cancer treatment method, and as a result, have found that, when expression of immune checkpoint protein and expression of lymphoid cell- or immune cell-specific protein are simultaneously checked and an immune checkpoint inhibitor is administered to a patient positive for expression of the immune checkpoint protein and negative for expression of the lymphoid cell- or immune cell-specific protein, the cancer therapeutic effect of the immune checkpoint inhibitor is enhanced because the immune checkpoint inhibitor can be administered only to patients responding to the immune checkpoint inhibitor while excluding false-positive patients, thereby completing the present invention.

SUMMARY OF THE INVENTION

An object of the present invention is to provide a method for selecting a patient for application of an immune checkpoint inhibitor.

Another object of the present invention is to provide a method for treating cancer.

Still another object of the present invention is to provide a method for determining susceptibility to an immune checkpoint inhibitor.

Yet another object of the present invention is to provide a method for selecting cancer immunotherapy for a cancer patient.

To achieve the above objects, the present invention provides a method for selecting a patient for application of a cancer immunotherapy drug, the method comprising steps of: (a) checking expression of immune checkpoint protein and lymphoid- or myeloid-specific protein in isolated circulating tumor cells; and (b) classifying, as a patient to whom the cancer immunotherapy drug is applicable, the patient found to be positive for expression of the immune checkpoint protein and negative for expression of the lymphoid- or myeloid-specific protein.

The present invention also provides a method for treating cancer comprising steps of: (a) checking expression of immune checkpoint protein and lymphoid- or myeloid-specific protein in isolated circulating tumor cells; and (b) administering an immune checkpoint inhibitor to a patient found to be positive for expression of the immune checkpoint protein and negative expression of the lymphoid- or myeloid-specific protein.

The present invention also provides a method for determining susceptibility to an immune checkpoint inhibitor, the method comprising steps of: (a) checking expression of immune checkpoint protein and lymphoid- or myeloid-specific protein in isolated circulating tumor cells; and (b) determining that the isolated circulating tumor cells are susceptible to the immune checkpoint inhibitor, when it is identified that expression of the immune checkpoint protein is positive and expression of the lymphoid- or myeloid-specific protein is negative.

The present invention also provides a method for selecting cancer immunotherapy for a cancer patient, the method comprising steps of: (a) checking expression of immune checkpoint protein and lymphoid- or myeloid-specific protein in isolated circulating tumor cells; and (b) selecting an immune checkpoint inhibitor as a therapeutic drug, when it is identified that expression of the immune checkpoint protein is positive and expression of the lymphoid- or myeloid-specific protein is negative.

The present invention also provides a pharmaceutical composition for treating cancer containing an immune checkpoint inhibitor for treating a subject positive for expression of immune checkpoint protein and negative for expression of lymphoid- or myeloid-specific protein.

The present invention also provides the use of an immune checkpoint inhibitor for the manufacture of a medicament for treating a subject positive for expression of immune checkpoint protein and negative for expression of lymphoid- or myeloid-specific protein.

BRIEF DESCRIPTION OF THE DRAWINGS

FIG. 1 shows fluorescence images of circulating tumor cells and immune cells, obtained according to an example of the present invention.

FIG. 2 shows fluorescence images of circulating tumor cells (A) expressing a circulating tumor cell marker and PD-L1, and false positive cells (myeloid cells, (B)) expressing both the marker and CD18, obtained according to an example of the present invention.

FIG. 3 depicts fluorescence images (A) showing the expression of CD16 and CD18 in myeloid cell lines, and graphs (B) showing the results of quantifying the expression, according to an example of the present invention.

FIG. 4 depicts fluorescence images (A) showing the expression of CD11b and CD18 in myeloid cell lines, and a graph (B) showing the results of quantifying the expression, according to an example of the present invention.

FIG. 5 depicts fluorescence images (A) showing the expression of CD16 and CD18 in patient's blood samples, and graphs (B) showing the results of quantifying the expression, according to an example of the present invention.

FIG. 6 shows the results of comparing the PD-L1 tumor proportion score between a tissue sample and CD18-/CD45-CTCs according to an embodiment of the present invention. (A): Bland-Altman plot; and (B): Scatter plot.

FIG. 7 shows the results of analyzing the correlation between the number of CTCs and the size of cancer tissue according to an example of the present invention.

DETAILED DESCRIPTION OF THE INVENTION

Unless otherwise defined, all technical and scientific terms used in the present specification have the same meanings as commonly understood by those skilled in the art to which the present disclosure pertains. In general, the nomenclature used in the present specification and the experimental methods described below are well known and commonly used in the art.

Terms, such as "first", "second", "A", "B", etc. can be used to describe various components, but the components are not limited by the terms. The terms are merely used to distinguish one component from another component. For example, the first component can be designated as the second component without departing from the scope of the present invention, and, similarly, the second component can also be designated as the first component. The term "and/or" includes a combination of a plurality of related listed items or any of a plurality of related listed items.

As used herein, singular expressions should be understood to include plural expressions unless otherwise specified in the context thereof. It should be understood that the terms "comprise", etc. are intended to denote the presence of stated features, numbers, steps, operations, components, parts, or combinations thereof, but do not exclude the presence or addition of one or more other features, numbers, steps, operations, components, parts, or combinations thereof.

Furthermore, in performing a method or operation method, steps constituting the method may be performed in order different from order described in the context unless the specific order is clearly described in the context. That is, the steps may be performed according to described order, or may be performed substantially at the same time, or may be performed in reverse order.

As used herein, the term "antibody" is used in the broadest sense and encompasses various antibody structures, including, but not limited to, monoclonal antibodies, polyclonal antibodies, multispecific antibodies (e.g., bispecific antibodies), and antibody fragments as long as they exhibit the desired antigen-binding activity.

As used herein, the term "biomarker" refers to an indicator can be detected in a sample. The biomarker may serve as an indicator of a particular disease or disorder (e.g., cancer) characterized by certain molecular, pathological, histological, and/or clinical features.

As used herein, the terms "cancer" and "cancerous" refer to or describe the physiological condition in mammals that is typically characterized by unregulated cell growth/proliferation. Examples of cancer include, but are not limited to, carcinoma, lymphoma, blastoma, sarcoma, and leukemia. More particular examples of such cancers include, but are not limited to, lung cancers, including small cell lung cancer, non-small cell lung cancer, lung adenocarcinoma, and squamous cell carcinoma of the lung; bladder cancers (e.g., urinary bladder cancer (UBC), muscle invasive bladder cancer (MIBC), and BCG-refractory non-muscle invasive bladder cancer (NMIBC)); kidney or renal cancer (e.g., renal cell carcinoma (RCC)); cancer of the urinary tract; breast cancer (e.g., HER2+ breast cancer and triple-negative breast cancer (TNBC), which are estrogen receptors (ER-), progesterone receptors (PR-), and HER2 (HER2-) negative); prostate cancer, such as castration-resistant prostate cancer (CRPC); cancer of the peritoneum; hepatocellular cancer;

gastric or stomach cancer, including gastrointestinal cancer and gastrointestinal stromal cancer; pancreatic cancer; glioblastoma; cervical cancer; ovarian cancer; liver cancer; hepatoma; colon cancer; rectal cancer; colorectal cancer; endometrial or uterine carcinoma; salivary gland carcinoma; prostate cancer; vulval cancer; thyroid cancer; hepatic carcinoma; anal carcinoma; penile carcinoma; melanoma, including superficial spreading melanoma, lentigo maligna melanoma, acral lentiginous melanomas, and nodular melanomas; multiple myeloma and B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); acute myelogenous leukemia (AML); hairy cell leukemia; chronic myeloblastic leukemia (CML); post-transplant lymphoproliferative disorder (PTLD); and myelodysplastic syndromes (MDS), as well as abnormal vascular proliferation associated with phacomatoses, edema (such as that associated with brain tumors), Meigs' syndrome, brain cancer, head and neck cancer, and associated metastases.

As used herein, the terms "treat," "treating" and "treatment" have their ordinary and customary meanings, and include one or more of complete or partial clearance of a tumor or cancer from a subject, reducing the size of a tumor in a subject, killing cells of a tumor or cancer in a subject, and ameliorating a symptom of cancer or a tumor in a subject. Treatment means clearing, reducing, killing or ameliorating by about 1% to about 100% versus a subject to whom an immune checkpoint inhibitor has not been administered. Preferably, the removing, reducing, killing or ameliorating is about 100%, about 99%, about 98%, about 97%, about 96%, about 95%, about 90%, about 80%, about 70%, about 60%, about 50%, about 40%, about 30%, about 20%, about 10%, about 5% or about 1%. The results of the treatment may be permanent or may continue for a period of days (such as 1, 2, 3, 4, 5, 6 or 7 days), weeks (such as 1, 2, 3 or 4 weeks), months (such as 1, 2, 3, 4, 5, 6 or more months), or years (such as 1, 2, 3, 4, 5, 6 or more years).

As used herein, the terms "inhibit", "inhibiting" and "inhibition" have their ordinary and customary meanings, and include one or more of, hindering, impeding, obstructing, deterring or restraining establishment of cancer or a tumor, development of cancer or a tumor, growth of cancer or a tumor and metastasis. Inhibition means hindering by about 1% to about 100% versus a subject to which an immune checkpoint inhibitor has not been administered. Preferably, the hindering is about 100%, about 99%, about 98%, about 97%, about 96%, about 95%, about 90%, about 80%, about 70%, about 60%, about 50%, about 40%, about 30%, about 20%, about 10%, about 5% or about 1%. The methods of inhibition may be practiced in a subject prior to, concurrent with, or after the onset of clinical symptoms of cancer or a tumor. Thus, the subject may have cancer or a tumor, or merely be susceptible to developing cancer or a tumor. The results of the inhibition may be permanent or may continue for a period of days (such as 1, 2, 3, 4, 5, 6 or 7 days), weeks (such as 1, 2, 3 or 4 weeks), months (such as 1, 2, 3, 4, 5, 6 or more months) or years (such as 1, 2, 3, 4, 5, 6 or more years).

As used herein, the term "administering" means a method of giving a dosage of a compound or a composition to a subject. The compound and/or composition used in the method described herein can be administered, for example, intravenously (e.g., by intravenous infusion), subcutaneously, intramuscularly, intradermally, percutaneously, intraarterially, intraperitoneally, intralesionally, intracranially, intraarticularly, intraprostatically, intrapleurally, intratracheally, intranasally, intravitreally, intravaginally, intrarectally, topically, intratumorally, peritoneally, subconjunctivally, intravesicularlly, mucosally, intrapericardially, intraumbilically, intraocularly, orally, topically, locally, by inhalation, by injection, by infusion, by continuous infusion, by localized perfusion bathing target cells directly, by catheter, by lavage, in cremes, or in lipid compositions. The method of administration can vary depending on various factors (e.g., the compound or composition being administered and the severity of the condition, disease, or disorder being treated).

The immune checkpoint inhibitor and a pharmaceutical formulation comprising the immune checkpoint inhibitor may be administered to a subject using different schedules, depending on the particular aim or goal of the method; the age and size of the subject; and the general health of the subject, to name only a few factors to be considered. In general, the immune checkpoint inhibitor and pharmaceutical formulation may be administered once, twice, three times, four times, five times, six times or more, over a course of treatment or inhibition. The timing between each dose in a dosing schedule may range between days, weeks, months, or years, and includes administered once every 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more weeks. The same quantity of the immune checkpoint inhibitor may be administered in each dose of the dosing schedule, or the amounts in each dose may vary. The identity of the immune checkpoint inhibitor may also vary or remain the same in each dose in a dosing schedule.

In each of the methods of the present invention, a "therapeutically effective amount" of an immune checkpoint inhibitor or pharmaceutical formulation comprising an immune checkpoint inhibitor is administered to a subject. The therapeutically effective amount will vary between subjects. However, the therapeutically effective amount is one that is sufficient to achieve the aim or goal of the method, whether inhibiting or treating. As an example, a therapeutically effective amount of an immune checkpoint inhibitor used in the method of the invention is typically between about 0.1 $\mu$g to about 10,000 $\mu$g of the immune checkpoint inhibitor per kg of body weight of the subject to which the peptide is administered. A therapeutically effective amount also includes about 0.5 $\mu$g to about 5000 $\mu$g, about 1 $\mu$g to about 500

µg, about 10 µg to about 200 µg, about 1 µg to about 800 µg, about 10 µg to about 1,000 µg, about 50 µg to about 5,000 µg, about 50 µg to about 500 µg, about 100 µg to about 1,000 µg, about 250 µg to about 2,500 µg, about 500 µg to about 2,000 µg, about 10 µg to about 800 µg, about 10 µg to about 1,000 µg, about 1 µg to about 300 µg, and about 10 µg to about 300 µg of the immune checkpoint inhibitor per kg of body weight of the subject.

In the case of Keytruda, which is a therapeutic drug used in existing immunotherapy, it was reported that, as a result of conducting the phase 2/3 'KEYNOTE-010' trial in patients expressing 1% or more of PD-L1, who had been treated with Keytruda, the therapeutic effect of Keytruda was better as the PD-L1 expression rate was higher, and the therapeutic response was relatively clearly differentiated depending on the PD-L1 expression rate, suggesting that PD-L1 is a good marker in cancer immunotherapy. However, in the case of Opdivo, it was reported that, as a result of retrospective analysis of the data from the phase 3 'Checkmate-057' trial in advanced non-squamous non-small cell lung cancer patients who had been treated with Opdivo, the objective response rates (ORRs) of the Opdivo-treated groups with PD-L1 expression rates of 1%, 5%, and 10% or more were 31%, 36% and 37%, respectively, which were similar.

In addition, it was reported that, in the 'OAK' trial of Tecentriq in patients with locally advanced or metastatic non-small cell lung cancer who failed existing platinum-based chemotherapy, the overall survival (OS) was prolonged by about 4 months compared to the existing chemotherapy docetaxel, irrespective of whether PD-L1 was expressed or not, suggesting that Tecentriq is effective not only in patients positive for PD-L1 expression but also in patients negative for PD-L1 expression.

It was reported that, in the phase 3 'PACIFIC' trial of Imfinzi in stage 3 non-small cell lung cancer patients who did not progress after simultaneous platinum-based chemotherapy and radiotherapy, the progression-free survival (PFS) was approximately doubled compared to a placebo group, and as a result of subgroup analysis, Imfinzi exhibited a consistent effect regardless of the PD-L1 expression rate.

After all, in the current state, even though PD-1/PD-L1 immune checkpoint inhibitors are administered, a dramatic therapeutic effect does not appear in all cancer patients, and there is a problem with the clinical usefulness of an immunohistochemistry (IHC) diagnostic device for measuring the PD-L1 expression rate for these therapeutic drugs.

For example, about 28% of patients who will respond to treatment are diagnosed with false negative PD-L1 expression and miss a chance to receive treatment, and about 42% of patients who will not respond to treatment are wrongly diagnosed with false positive PD-L1 expression, and thus there is a risk that the appropriateness and economic feasibility of drug administration may be degraded.

The present inventors have made extensive efforts to develop a biomarker capable of eliminating false-positive cells with high efficiency and develop a method for effectively selecting patients to whom cancer immunotherapy is applicable, and as a result, have found that, when the expression of immune checkpoint protein and the expression of lymphoid- or myeloid-specific protein are simultaneously checked, cancer immunotherapy can be selected with high accuracy.

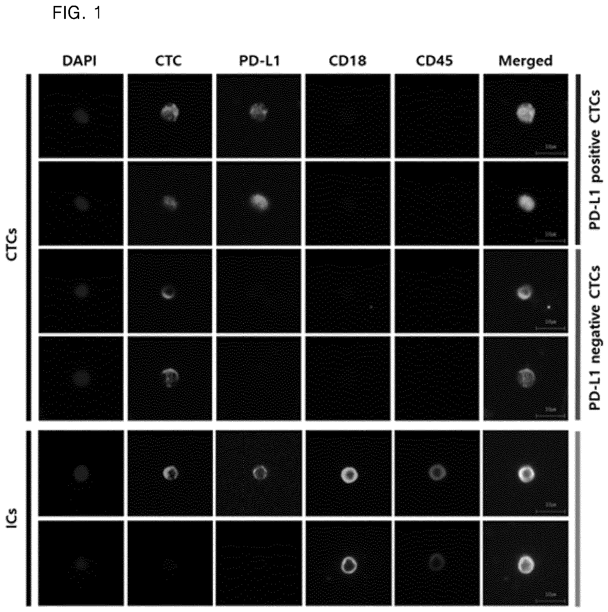

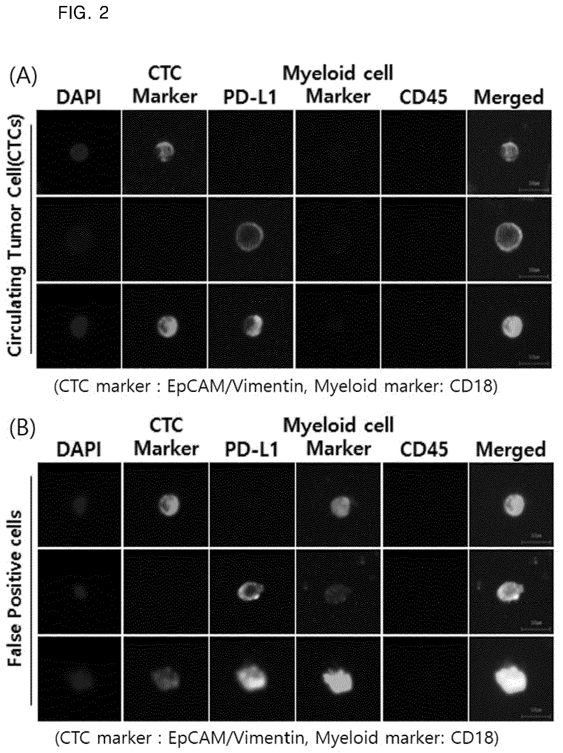

That is, in one example of the present invention, it was confirmed that, when CTCs isolated from blood were stained with PD-L1 and CD18 antibodies and the expression levels of these proteins were checked, false-positive cells could be effectively eliminated compared to the method of checking only the expression of PD-L1 (FIGS. 1 and 2).

Therefore, in one aspect, the present invention is directed to a method for selecting a patient for application of a cancer immunotherapy drug, the method comprising steps of: (a) checking expression of immune checkpoint protein and lymphoid- or myeloid-specific protein in isolated circulating tumor cells; and (b) classifying, as a patient to whom the cancer immunotherapy drug is applicable, the patient found to be positive for expression of the immune checkpoint protein and negative for expression of the lymphoid- or myeloid-specific protein.

In the present invention, the expression "protein expression is positive" means that the presence of the protein in isolated circulating tumor cells is identified by various methods known to those skilled in the art, preferably by fluorescence intensity, without being limited thereto.

In the present invention, determining that the expression of the protein of interest is positive may be based on a specific cut-off value in consideration of the number of cells in which each or all of the proteins are expressed, and on the expression intensity (fluorescence intensity) of each protein, or may be made by determining whether the expression levels are higher or lower than those in a reference population, without being limited thereto.

In the present invention, the expression "protein expression is negative" means that the presence of the protein in isolated circulating tumor cells is not identified by various methods known to those skilled in the art, preferably by fluorescence intensity, without being limited thereto.

In the present invention, determining that the expression of the protein of interest is negative may be based on a specific cut-off value in consideration of the number of cells in which each or all of the proteins are not expressed, and on the expression intensity (fluorescence intensity) of each protein, or may be made by determining whether the expression levels are higher or lower than those in a reference population, without being limited thereto.

In the present invention, the immune checkpoint protein may be, without limitation, any protein involved in immune checkpoint-related signaling pathways. Preferably, the immune checkpoint protein may be selected from the group consisting of CD27, CD28, CD40, CD122, CD137, OX40, GITR, ICOS, A2AR, B7-H3, BY-H4, CTLA-4, IDO, KIR, LAG3, NOX2, PD-1, PD-L1, TIM-3, VISTA, and SIGLEC7, and more preferably, selected from the group consisting of PD-1, PD-L1 and CTLA-4, with PD-L1 being most preferred, without being limited thereto.

In the present invention, the lymphoid- or myeloid-specific protein may be, without limitation, any protein expressed in lymphoid or myeloid cells. Preferably, the lymphoid- or myeloid-specific protein may be selected from the group consisting of CD18, CD29, CD61, CD104, ITGB5, ITGB6, ITGB7, ITGB8, CD49a, CD49b, CD49c, CD49d, CD49e, CD49f, ITGA7, ITGA8, ITGA9, ITGA10, ITGA11, CD11D, CD103, CD11a, CD11b, CD51, CD41, CD11c, CD64, CD32, CD16a, CD16b, CD23, CD89, FcRn, FcεRI, and Fcα/μR, and more preferably, may be selected from the group consisting of CD18, CD16, CD29, CD61, CD104, CD32, CD11b and CD64, with CD18 being most preferred, without being limited thereto.

In the present invention, the isolated circulating tumor cells may be obtained by performing the following steps:

(i) obtaining blood from a cancer patient; and
(ii) isolating circulating tumor cells from the blood using a biochip.

As used herein, the term "circulating tumor cells (CTCs)" refers to tumor cells found in the peripheral blood of malignant tumor patients. Circulating tumor cells are very rare and the amount of samples available is very limited. Techniques for the detection and characterization of circulating cancer cells in blood include, but are not limited to, multiplex reverse transcriptase-quantitative polymerase chain reaction methods, imaging-based approaches, microfiltration techniques, and microporous chip devices. The circulating tumor cells are liquid biopsy samples that can act as tumor biomarkers that can inevitably provide individualized treatment and post-treatment follow-up. In addition, the circulating tumor cells may be used as targets for understanding the biological characteristics of tumors and the seeding of tumor cells, without being limited thereto.

The biochip is a hybrid device fabricated in an existing semiconductor chip form by highly integrating and combining substances, such as organism-derived DNA, proteins, enzymes, antibodies, microorganisms, animal and plant cells and organs, and neurons, on a solid substrate made of an inorganic material such as a semiconductor. The biochip refers to a tool or device that uses the inherent functions of biomolecules to obtain biological information, such as gene expression patterns, gene binding or protein distributions, or speeds up biochemical processes and reactions or information processing.

The high-density microporous chip refers to a biochip capable of isolating a substance having a specific size based on the working principle of the biochip.

The high-density microporous chip is based on the size difference of blood cells and can capture circulating tumor cells with a recovery rate of about 90% within 10 minutes.

In one embodiment of the present invention, the pore size of the high-density microporous chip may preferably be 5.5 to 8.5 μm, more preferably 6.5 to 7.5 um. If the pore size is smaller than 5.5 um, red blood cells and white blood cells cannot be removed, because these cells remain on the chip without passing through the chip, and if the pore size is larger than 8.5 um, circulating tumor cells cannot be selectively recovered, because the pore size is larger than the size of the circulating tumor cells and thus the circulating tumor cells pass through the chip. In one embodiment of the present invention, the pore shape of the high-density microporous chip may be circular, rectangular or elliptical, preferably rectangular.

In one embodiment of the present invention, the pores of the high-density microporous chip may be arranged in a regular pattern. In another embodiment of the present invention, the high-density microporous chip may be specifically made of stainless steel, nickel, aluminum, or copper. The pores may be formed by etching using MEMS (Microelectromechanical Systems) technology.

The spacing between two adjacent pores of the pores is narrower than the diameter of circulating tumor cells. According to one embodiment of the invention, the spacing between the two pores may be 45 to 65% relative to the diameter of circulating tumor cells. The high-density microporous chip is not deformed by the pressure of the blood or solution flowing through the channel. After passing through the pores, the blood is discharged to the outside and the circulating tumor cells in the blood remain on the surface of the chip without passing through the pores.

Non-target cells, that is, red blood cells having a higher deformation rate than the circulating tumor cells, easily pass through the pores.

After filtration of the circulating tumor cells, the circulating tumor cells may be discharged to the outside by supplying a solution in the reverse or forward direction. According to one embodiment of the present invention, the solution may be supplied in the reverse direction so as to minimize damage to the circulating tumor cells. The solution may be supplied by a syringe, a syringe pump, a plunger pump, or the like. According to one embodiment of the present invention, the solution may be composed of a diluent, water and diluent acid, which dilute the blood. The circulating tumor cells discharged to the outside by supplying the solution may be easily collected in a container, for example, a test tube or a culture dish.

Meanwhile, circulating tumor cells having a diameter of 7.5 to 15 um pass through a tube having a diameter of 8 um when a pressure of about 100 mmHg is applied thereto. Circulating tumor cells having a diameter of 15 $\mu$m, which pass through the 8-$\mu$m diameter tube, have a deformation rate of about 53%. The spacing between two pores is preferably 4 um or less in consideration of the deformation rate of the circulating tumor cells when the circulating tumor cells having a diameter of 7.5 um are discharged to the outside by back washing, i.e., allowing the solution to flow in a direction opposite to the flow of the blood. Non-small cell lung cancer and breast cancer cells, for example, are known to have a diameter reaching about 40 um. In consideration of the deformation rate of circulating cancer cells having a diameter of 40 um during back washing, the spacing between two pores is preferably set to 21 um or less. When circulating cancer cells having a diameter of 7.5 um are present between two pores having a spacing larger than 4 $\mu$m, the circulating tumor cells may not be detached from the surface as they are deformed by the flow of the solution. In addition, tumor cells having a diameter of 40 um may also not be detached from the surface between two pores having a spacing larger than 21 um. In the high-density microporous chip according to the present invention, the spacing between two pores is 45 to 65% of the diameter of the circulating tumor cells in consideration of the pressure of the solution. If the spacing is larger than 65%, that is, larger than about 4.9 $\mu$m, circulating tumor cancer cells having a diameter of 7.5 um may not be detached from the surface between two pores by back washing. If the spacing is larger than 65%, that is, larger than 26 $\mu$m, circulating tumor cells having a diameter of 40 um may not be detached from the surface between two pores by back washing. If the pressure of the solution is increased to forcibly detach the circulating tumor cells attached to the surface between two pores, damage to the circulating tumor cells may occur, which reduces the collection rate of living circulating tumor cells. If the spacing for circulating cancer cells having a diameter of 7.5 um is less than 45%, that is, smaller than about 3.375 um, the high-density microporous chip is highly likely to be damaged by the flow of the blood and the solution.

In one embodiment of the present invention, the sample may be repeatedly passed through the high-density microporous chip. Specifically, after circulating tumor cells are isolated once from the high-density microporous chip, the isolated circulating tumor cells may be loaded again on the high-density microporous chip and isolated, and the isolation process may be repeated.

In one embodiment of the present invention, isolation of the circulating tumor cells through the high-density microporous chip is not performed by applying a specific artificial pressure after loading the solution containing the circulating tumor cells onto the high-density microporous chip, but may be performed using gravity. Isolation of circulating tumor cells through the high-density microporous chip according to the present invention may minimize damage caused to the circulating tumor cells by artificial pressure, thereby maintaining the circulating tumor cells in the same state as when these cells are present in the body of the patient.

In one embodiment of the present invention, the high-density microporous chip may be coated with a specific material in order to minimize damage caused to the circulating tumor cells by the high-density microporous chip during isolation of the circulating tumor cancer cells, or to make repeated use of the high-density microporous chip more efficient, or to make recovery of the circulating tumor cells more efficient. Specifically, the specific material may be an antibody that can bind specifically to the circulating tumor cells, and may also be any biomaterial that does not physically or chemically damage the cells. According to one embodiment of the present invention, the specific material may be BSA (bovine serum albumin) or an antibody. The antibody may be composed of, for example, an anti-epithelial cell adhesion molecule antibody (anti-EpCAM antibody), an anti-cytokeratin antibody (anti-CK antibody), or the like. According to a preferred embodiment of the present invention, the specific material may be BSA (bovine serum albumin).

The BSA (bovine serum albumin) solution refers to bovine serum albumin. It is a protein having a molecular weight of about 66.4 kDa, which is abundantly found in most animals. The BSA may be added as a nutrient to cells during cell culture in biochemistry/biology, and is also frequently used as a standard for obtaining a calibration curve in protein quantification. In addition, since a small amount of enzyme (protein) needs to be used when a restriction enzyme is used, the BSA may also be added to supplement the concentration of the protein in a solution. In addition, in various biochemical experiments (Western blotting, immunocytochemistry, ELISA, etc.), the BSA may also be used to prevent nonspecific binding, that is, prevent a specific antibody from binding to an unwanted protein or an unwanted position, before the specific antibody is attached to a protein to be detected.

According to one embodiment of the present invention, centrifugation may be used to allow peripheral blood to react with the high-density microporous chip coated with the BSA solution, thereby removing biopolymers other than the circulating tumor cells. According to one embodiment of the present invention, isolation of the circulating tumor cells using the high-density microporous chip may be performed using gravity. Specifically, isolation may be performed under a pressure of atmospheric pressure (1,000 hPa) to 1,020 hPa. Preferably, it may be performed under a pressure of atmospheric pressure (1,000 hPa) to 1,015 hPa. More preferably, it may be performed under a pressure of atmospheric pressure (1,000 hPa) to 1,013 hPa.

According to one embodiment of the present invention, the upper surface or lower surface of the high-density microporous chip or the inner surface of the pores may be coated with the BSA solution. Preferably, not only the upper surface and lower surface of the high-density microporous chip, but also the inner surface of the pores may be coated with the BSA solution.

According to one embodiment of the present invention, coating with the BSA solution may be performed at a BSA concentration of 0.05 to 0.15%. According to a preferred embodiment of the present invention, coating with the BSA solution may be performed at a BSA concentration of 0.08 to 0.012%.

According to one embodiment of the present invention, coating with the BSA solution may be performed for 5 to 15 minutes. According to one preferred embodiment of the present invention, coating with the BSA solution may be performed for 8 to 12 minutes.

In the present invention, expression of the immune checkpoint protein and the lymphoid- or myeloid-specific protein may be checked by performing the following steps:

(1) allowing circulating tumor cells to react with a fluorescent marker that binds specifically to the circulating tumor cells, a fluorescent marker that binds specifically to the immune checkpoint protein, and a fluorescent marker that binds specifically to the lymphoid- or myeloid-specific protein;
(2) receiving optical images of the circulating tumor cells reacted with the fluorescent marker, the immune checkpoint protein, and the lymphoid- or myeloid-specific protein in a plurality of wavelength ranges, respectively;
(3) performing first filtering by measuring fluorescence intensities of the circulating tumor cells, the immune checkpoint protein, and the lymphoid- or myeloid-specific protein in the optical images in all or part of the plurality of wavelength ranges;
(4) performing second filtering by measuring morphology of the circulating tumor cells in the optical images in all or part of the plurality of wavelength ranges; and
(5) performing third filtering by measuring morphology of the circulating tumor cells in a combined image obtained by merging all or part of the optical images in the plurality of respective wavelength ranges.

In the present invention, the fluorescent marker that binds specifically to the circulating tumor cells refers to a fluorescent substance that may bind specifically to the circulating tumor cells themselves or to substances present inside or outside the circulating tumor cells. According to one embodiment of the present invention, the fluorescent marker capable of identifying the circulating tumor cells may bind specifically to the cell nucleus or bind specifically to the protein, DNA, RNA or the like present inside or outside the cells. Specifically, for binding to the cell nucleus, DAPI may be used as the fluorescent marker. Furthermore, a fluorescent marker that binds specifically to vimentin, an intermediate filament protein, may be used. In addition, a fluorescent marker that binds specifically to epithelial cell adhesion molecule (EpCAM) and cytokeratin (CK) may be used, and a fluorescent marker may also be used, which binds specifically to CD45 which is used as a means for removing non-target cells. The fluorescent marker that binds specifically to the circulating tumor cells may be composed of a nucleotide, an oligonucleotide, a peptide, a polypeptide, a nucleic acid or a protein, and may preferably be an antibody composed of protein. The fluorescent marker that binds specifically to the circulating tumor cells may be any substance that makes it possible to identify the circulating tumor cells by specific binding to the cells.

The optical images may be generated by an imaging system. According to one embodiment of the present invention, the imaging system may be a cell imaging system. The cell imaging system is a system configured to place stained cells on a platform such as a glass slide and observe and image the cells at various wavelengths. The cell imaging system may include an automated cell counting module, a fluorescence intensity analysis module, and a cytology-based cell classification/cognition module. In addition, as user convenience functions, a measurement function, a report automatic generation function and a DB management function may be included as user interfaces. This cell imaging system includes digital image analysis equipment for distinguishing cells, culture media, debris, and the like, and for identifying and counting desired cells by a user. The cell imaging system according to one embodiment of the present invention can accurately identify and count fluorescently labeled or marker-bound target cells from optical images.

The optical images may be optical images obtained by the light reflected from an object. The optical images of the cells may be output by the cell imaging system and may include images of cells, debris and the like on the background. Such optical images may be provided as a single image file constructed by stitching together a plurality of divided images for the respective specific wavelength ranges. Here, the optical images in the plurality of wavelength ranges may include a blue wavelength range image, a green wavelength range image, and a red wavelength range image. The optical image for the blue wavelength range is particularly useful for the identification of the nucleus of the circulating tumor cells, and the optical images for the green and red wavelength ranges are particularly useful for the identification of the membrane of the circulating tumor cells. In addition, it is possible to identify specific fluorescent probes or markers by using the wavelength ranges of various colors.

In the first filtering or the second filtering, if the identification of the circulating tumor cells, the immune checkpoint protein or the lymphoid cell- or myeloid cell-specific protein is not achieved at a desired level, it is possible to perform filtering of the optical image data and perform the first filtering or the second filtering once again, and it is also possible to perform data filtering a plurality of times. In addition, after the first filtering or the second filtering is performed, the image data may be stored and output.

Furthermore, after performing the first filtering step and the second filtering step on the optical image for the first wavelength range, one or more of the first filtering step and the second filtering step on the optical image for the second wavelength range different from the first wavelength range may further be performed.

For example, the first wavelength range may be a blue wavelength range, and the second wavelength range may be a green or red wavelength range. In this case, the nucleus of the circulating tumor cells may be identified by performing the first filtering process and the second filtering process on the blue wavelength range image. In addition, the cell membrane of the circulating tumor cells may be identified by performing a first filtering process on one or more of the green wavelength range image and the red wavelength range image. These first and second filtering processes make it possible to accurately identify the cells from the optical image. For example, the green and red wavelength range images increase identification of target cells, for example, white blood cells and tumor cells.

Meanwhile, in the second filtering process, the morphology of the circulating tumor cells is measured. Here, the morphology of the circulating tumor cells may include one or more of cell area, cell size (diameter), and cell circularity.

In one embodiment of the invention, measurement of the fluorescence intensities of the immune checkpoint protein and the lymphoid cell- or myeloid cell-specific protein is performed by measuring the fluorescence intensities on the optical images of all or part of a plurality of wavelength ranges resulting from the fluorescence markers that bind specifically to the lymphoid cell- or myeloid cell-specific protein, and first filtering thereof may be performed.

The process of performing the first filtering by measuring the fluorescence intensity of the circulating tumor cells other than the immune checkpoint protein and the lymphoid cell- or myeloid cell-specific protein will now be described in more detail. The first filtering may be performed by measuring the cell size in an optical image of all or part of a plurality of wavelength ranges, and then setting a polygonal or circular area, which is larger than the measured cell size by a predetermined ratio or amount, and measuring the fluorescence intensity of the cells within this area.

In the third filtering step, the third filtering is performed by measuring the morphology of the circulating tumor cells in a combined image obtained by merging all or part of the optical images in the plurality of wavelength ranges. Here, the morphology of the circulating tumor cells may include one or more of cell area, cell size, and cell circularity.

The third filtering may be performed on the whole circulating tumor cancer cells, and may also be additionally performed on the cells which have been difficult to identify in the first and second filtering processes. If additional identification is required, separate data filtering may be performed or the third filtering may also be performed multiple times.

The image analysis may be executed by a computer program. This computer program may be implemented using one or more general-purpose or special-purpose computers, such as a processor, a controller, an arithmetic logic unit (ALU), a digital signal processor, a microcomputer, a field programmable gate array (FPGA), a programmable logic unit (PLU), a microprocessor, or any other devices capable of executing and responding to instructions.

In the present invention, the fluorescent marker that binds specifically to the immune checkpoint protein may be selected from the group consisting of an antibody specific to PD-1, an antibody specific to PD-L1, and an antibody specific to CTLA-4, without being limited thereto.

In the present invention, the fluorescent marker that binds specifically to the lymphoid- or myeloid-specific protein may be selected from the group consisting of an antibody specific to CD18, an antibody specific to CD16, an antibody specific to CD29, an antibody specific to CD61, an antibody specific to CD104, an antibody specific to CD32, an antibody specific to CD11b, and an antibody specific to CD64, without being limited thereto.

In the present invention, the immune checkpoint inhibitor may be, without limitation, any substance capable of inhibiting the function of the immune checkpoint protein. Specifically, it may be a protein, a compound, a natural product, DNA, RNA, a peptide, or the like, preferably an antibody, more preferably a monoclonal antibody, even more preferably a human antibody, a humanized antibody, or a chimeric antibody.

In the present invention, the immune checkpoint inhibitor may be any one or more selected from the group consisting of a PD-L1 antagonist, a PD-1 antagonist, and a CTLA-4 antagonist, without being limited thereto.

As used herein, the term "PD-1 antagonist" refers to a molecule that decreases, blocks, inhibits, abrogates or interferes with signal transduction resulting from the interaction of PD-1 with one or more of its binding partners, such as PD-L1 and/or PD-L2. In some embodiments, the PD-1 antagonist is a molecule that inhibits the binding of PD-1 to its binding partners. In a specific aspect, the PD-1 antagonist inhibits the binding of PD-1 to PD-L1 and/or PD-L2. For example, PD-1 antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with its binding partner, such as PD-L1 or PD-L2. In one embodiment, the PD-1 antagonist reduces the negative co-stimulatory signal mediated by or through cell surface proteins expressed on T lymphocytes mediated signaling through PD-1 so as render a dysfunctional T-cell less dysfunctional. In some embodiments, the PD-1 antagonist is an anti-PD-1 antibody.

As used herein, the term "PD-L1 antagonist" refers to a molecule that decreases, blocks, inhibits, abrogates or interferes with signal transduction resulting from the interaction of PD-L1 with either one or more of its binding partners, such as PD-1 or B7-1. In some embodiments, the PD-L1 antagonist is a molecule that inhibits the binding of PD-L1 to its binding partners. In a specific aspect, the PD-L1 antagonist inhibits binding of PD-L1 to PD-1 and/or B7-1. In some embodiments, the PD-L1 antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with its binding partner, such as PD-1 or B7-1. In one embodiment, the PD-L1 antagonist reduces the negative co-stimulatory signal mediated by or through cell surface proteins expressed on T lymphocytes mediated signaling through PD-L1 so as to render a dysfunctional T cell less dysfunctional (e.g., enhancing effector responses to antigen recognition). In some embodiments, the PD-L1 antagonist is an anti-PD-L1 antibody.

As used herein, the term "CTLA4 antagonist" refers to a molecule that decreases, blocks, inhibits, abrogates or interferes with signal transduction resulting from the interaction of CTLA4 with one or more of its binding partners, such as B7-1. In some embodiments, the CTLA4 antagonist is a molecule that inhibits the binding of CTLA4 to its binding partners. In a specific aspect, the CTLA4 antagonist inhibits the binding of CTLA4 to B7-1. In some embodiments, the CTLA4 antagonists include anti-CTLA4 antibodies, antigen binding fragments thereof, immunoadhesins, fusion proteins, oligopeptides and other molecules that decrease, block, inhibit, abrogate or interfere with signal transduction resulting from the interaction of CTLA4 with its binding partners, such as B7-1. In one embodiment, the CTLA4 antagonist reduces the negative co-stimulatory signal mediated by or through cell surface proteins expressed on T lymphocytes mediated signaling through CTLA4 so as to render a dysfunctional T cell less dysfunctional (e.g., enhancing effector responses to antigen recognition). In some embodiments, the CTLA4 antagonist is an anti-CTLA4 antibody.

In the present invention, the immune checkpoint inhibitor may be at least one selected from the group consisting of pembrolizumab (Keytruda), nivolumab (Opdivo), cemiplimab (Lybtayo), atezolizumab (Tecentriq), avelumab (Bacencio), durvalumab (Imfinzi), ipilimumab (Yervoy), and tremelimumab, without being limited thereto.

In the present invention, the cancer may be selected from the group consisting of lung cancer, kidney cancer, bladder cancer, breast cancer, colorectal cancer, ovarian cancer, pancreatic cancer, gastric carcinoma, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycosis fungoides, Merkel cell carcinoma, and hematologic malignancies, and may preferably be lung cancer, most preferably non-small cell lung cancer, without being limited thereto.

In another aspect, the present invention is directed to a method for treating cancer comprising steps of: (a) checking expression of immune checkpoint protein and lymphoid- or myeloid-specific protein in isolated circulating tumor cells; and (b) administering an immune checkpoint inhibitor, when expression of the immune checkpoint protein is positive and expression of the lymphoid- or myeloid-specific protein is negative.

In another aspect, the present invention is directed to a method for determining susceptibility to an immune checkpoint inhibitor, the method comprising steps of: (a) checking expression of immune checkpoint protein and lymphoid- or myeloid-specific protein in isolated circulating tumor cells; and (b) determining that the circulating tumor cells are susceptible to the immune checkpoint inhibitor, when expression of the immune checkpoint protein is positive and expression of the lymphoid- or myeloid-specific protein is negative.

In another aspect, the present invention is directed to a method for selecting cancer immunotherapy for a cancer patient, the method comprising steps of: (a) checking expression of immune checkpoint protein and lymphoid- or myeloid-specific protein in isolated circulating tumor cells; and (b) selecting an immune checkpoint inhibitor as a therapeutic drug, when it is identified that expression of the immune checkpoint protein is positive and expression of the lymphoid- or myeloid-specific protein is negative.

In another aspect, the present invention is directed to a pharmaceutical composition for treating cancer containing an immune checkpoint inhibitor for treating a subject positive for expression of immune checkpoint protein and negative for expression of lymphoid- or myeloid-specific protein.

In another aspect, the present invention is directed to the use of an immune checkpoint inhibitor for the manufacture of a medicament for treating a subject positive for expression of immune checkpoint protein and negative for expression of lymphoid- or myeloid-specific protein.

## Examples

Hereinafter, the present invention will be described in more detail with reference to examples. These examples are only for illustrating the present invention, and it will be obvious to those of ordinary skill in the art that the scope of the present invention is not to be construed as being limited by these examples.

## Statistical Analysis

Data from patients were used to compare baseline and demographic characteristics. All values are expressed as mean $\pm$ standard deviation or as numbers (percentages). T-test was used to compare continuous variables, and Chi-square test was used to compare categorical variables. Accuracy and weighted kappa were used to compare PD-L1 positivity on tissue samples and blood samples. Concordance of PD-L1 expression as a continuous variable between CTCs and tissue samples was analyzed using Bland-Altman plots. The relationship between tumor burden and the number of CTCs was analyzed using the Pearson correlation coefficient. Baseline clinical tumor burden was determined using the sum of the longest diameters of target lesions (a maximum of 2 per organ and 5 in total) according to version 1.1 of the response assessment criteria in solid tumors on CT. A p-value of less than 0.05 was considered statistically significant. The statistical analysis was performed in IBM SPSS version 25.0 (IBM Corp., Armonk, NY, USA).

## Example 1. Isolation of CTCs from Blood

This study was approved by the Research Ethics Committee (IRB), and tissue and blood samples from 29 lung cancer patients (Table 1) and 30 infected patients (Table 2) were used after written consent.

[Table 1] Information on each lung cancer patient

| Patient number | Age | Sex | Diagnosis | Stage | EGFR | ALK | PD-L1 expression (%) |
|---|---|---|---|---|---|---|---|
| 1 | 59 | Male | Adenocarcinoma | 4 | Not done | Not done | 30 |
| 2 | 59 | Female | Adenocarcinoma | 4 | Not done | Mutation | 0 |
| 3 | 68 | Male | Adenocarcinoma | 4 | Not done | Not done | 1 |
| 4 | 65 | Male | Adenocarcinoma | 4 | Not done | Not done | 100 |
| 5 | 52 | Male | Adenocarcinoma | 4 | Mutation | Not done | 0 |
| 6 | 71 | Female | NSCLC NOS | 3 | Mutation | Not done | 100 |
| 7 | 42 | Male | Adenocarcinoma | 4 | Mutation | Not done | 0 |
| 8 | 74 | Male | Squamous cell carcinoma | 4 | Not done | Not done | 90 |
| 9 | 62 | Male | Squamous cell carcinoma | 3 | Wild type | Wild type | 95 |
| 10 | 68 | Male | Adenocarcinoma | 4 | Mutation | Not done | 1 |
| 11 | 59 | Male | Adenocarcinoma | 4 | Not done | Not done | 1 |
| 12 | 55 | Male | Adenocarcinoma | 3 | Wild type | Wild type | 0 |
| 13 | 62 | Female | Adenocarcinoma | 4 | Not done | Not done | 0 |
| 14 | 54 | Female | Adenocarcinoma | 4 | Not done | Not done | 80 |
| 15 | 47 | Male | Adenocarcinoma | 4 | Not done | Not done | 0 |
| 16 | 74 | Female | Adenocarcinoma | 4 | Mutation | Not done | 5 |
| 17 | 75 | Male | Squamous cell carcinoma | 3 | Wild type | Wild type | 5 |
| 18 | 67 | Male | Adenocarcinoma | 4 | Not done | Not done | 0 |
| 19 | 70 | Male | Adenocarcinoma | 4 | Mutation | Not done | 90 |
| 20 | 66 | Male | Adenocarcinoma | 4 | Not done | Not done | 0 |
| 21 | 58 | Male | Adenocarcinoma | 4 | Not done | Not done | 0 |
| 22 | 67 | Male | Squamous cell carcinoma | 4 | Wild type | Not done | 0 |
| 23 | 58 | Male | Adenocarcinoma | 4 | Not done | Not done | 30 |
| 24 | 77 | Male | Squamous cell carcinoma | 4 | Wild type | Wild type | 0 |
| 25 | 68 | Female | Squamous cell carcinoma | 4 | Wild type | Wild type | 70 |
| 26 | 55 | Female | Adenocarcinoma | 4 | Mutation | Not done | 5 |
| 27 | 63 | Male | Squamous cell carcinoma | 3 | Wild type | Wild type | 60 |
| 28 | 70 | Male | Squamous cell carcinoma | 3 | Wild type | Wild type | 90 |
| 29 | 56 | Male | Adenocarcinoma | 3 | Mutation | Wild type | 0 |

(EGFR, epidermal growth factor receptor; ALK, anaplastic lymphoma kinase; PD-L1, programmed cell death-ligand 1; NSCLC, non-small cell lung cancer; NOS, not otherwise specified)

[Table 2] Information on each infected patient

| Patient number | Age | Sex | Diagnosis |
|---|---|---|---|
| 1 | 61 | Female | Bacterial pneumonia |
| 2 | 53 | Female | NTM disease |

(continued)

| Patient number | Age | Sex | Diagnosis |
|:---:|:---:|:---:|:---:|
| 3 | 66 | Male | Tuberculosis |
| 4 | 60 | Male | Bacterial pneumonia |
| 5 | 54 | Male | Parasitic infection |
| 6 | 66 | Female | Focal pneumonia, organism unspecified |
| 7 | 78 | Female | Focal pneumonia, organism unspecified |
| 8 | 71 | Male | NTM disease |
| 9 | 59 | Female | Focal pneumonia, organism unspecified |
| 10 | 69 | Male | Focal pneumonia, organism unspecified |
| 11 | 66 | Male | Focal pneumonia, organism unspecified |
| 12 | 54 | Male | Tuberculosis |
| 13 | 68 | Female | Bacterial pneumonia |
| 14 | 59 | Female | NTM disease |
| 15 | 46 | Male | Focal pneumonia, organism unspecified |
| 16 | 59 | Female | NTM disease |
| 17 | 38 | Male | Bacterial pneumonia |
| 18 | 56 | Female | NTM disease |
| 19 | 60 | Male | Tuberculosis |
| 20 | 65 | Male | NTM disease |
| 21 | 55 | Female | Fungal pneumonia |
| 22 | 45 | Male | Focal pneumonia, organism unspecified |
| 23 | 66 | Female | NTM disease |
| 24 | 42 | Female | Tuberculosis |
| 25 | 63 | Male | Focal pneumonia, organism unspecified |
| 26 | 73 | Male | Focal pneumonia, organism unspecified |
| 27 | 69 | Male | Focal pneumonia, organism unspecified |
| 28 | 64 | Female | Bacterial pneumonia |
| 29 | 41 | Male | Tuberculosis |
| 30 | 59 | Female | Bacterial pneumonia |

(NTM: Nontuberculous Mycobacterial)

Table 3 below summarizes basic information on the patients.

[Table 3] Summary of information on lung cancer patients and infected patients

| | Total (n = 59) | Lung cancer group (n = 29) | Infection group (n = 30) |
|:---:|:---:|:---:|:---:|
| Age | 61.1 ± 9.3 | 62.8 ± 8.5 | 59.5 ± 9.8 |
| Male sex | 38 (64.4) | 22 (75.9) | 16 (53.3) |
| Ever-smoker | 43 (72.9) | 21 (72.4) | 11 (36.7) |
| Type of pathology | n = 29 | | |
| Adenocarcinoma | | 20 (69.0) | - |
| Squamous cell carcinoma | | 8 (27.6) | - |
| NSCLC, NOS | | 1 (3.4) | - |
| Stage of lung cancer | n = 29 | | |
| Stage III | | 7 (24.1) | - |
| Stage IV | | 22 (75.9) | - |
| Type of infection | n = 30 | | |
| Bacteria | | - | 6 (20.0) |
| Tuberculosis | | - | 5 (16.7) |
| NTM | | - | 7 (23.3) |
| Fungus | | - | 1 (3.3) |

(continued)

|  | Total (n = 59) | Lung cancer group (n = 29) | Infection group (n = 30) |
|---|---|---|---|
| Parasite | | - | 1 (3.3) |
| Focal pneumonia, organism unspecified | | - | 10 (33.3) |

The values are mean $\pm$ standard deviation (SD) or numbers (%)
(NSCLC, non-small cell lung cancer; NOS, not otherwise specified; SCLC, small cell lung cancer; NTM, nontuberculous mycobacteria)

In addition, Table 4 below summarizes molecular diagnostic information related to PD-L1 expression obtained during recruitment of the lung cancer patient group.

[Table 4]

Results of analysis of tissue samples from lung cancer group

|  | Lung cancer group (n = 29) |
|---|---|
| EGFR | |
| Mutation | 8 (27.6) |
| Wild type | 8 (27.6) |
| Unknown | 13 (44.8) |
| ALK | |
| Mutation | 8 (27.6) |
| Wild type | 1 (3.4) |
| Unknown | 20 (69.0) |
| PD-L1 expression | |
| $\geq$ 50% | 9 (31.0) |
| 1 - 49% | 8 (27.6) |
| <1% | 12 (41.4) |

(EGFR, epidermal growth factor receptor; ALK, anaplastic lymphoma kinase; PD-L1, programmed cell death-ligand 1)

CTCs were isolated from the patient's blood using SmartBiopsy™ cell isolation kit (CytoGen) and SmartBiopsy™ cell isolator (CytoGen) according to the manufacturer's instructions. As a high-density microporous chip applied to the SmartBiopsy™ cell isolator, a chip with a pore size of 5 um was used so that target cells larger than 5 um were collected on the chip. Since the pore size of the microporous chip is adjustable, the microporous chip is a microporous chip designed to selectively recover cells having a specific size (US10,502,668, and KR10-1254675).

**Smart Biopsy™ Cell Isolator (CIS030)**

- Smart Biopsy™ Cell Isolator is a system that recognizes the height of target cells (peripheral blood mononuclear cells, PBMCs) from a solution and automatically performs mixing, transport and filtering using an air displacement pipette (ADP). This system consists of main X-Y axes, ADP, a chip handler, and a consumable table. Basically, Smart Biopsy™ Cell Isolator is operated according to the user manual using software.

**Example 2. Selection of markers for false-positive filtering of isolated CTCs**

2-1. CTC staining method

**Smart Biopsy™ IF Stainer (CST030)**

- Smart Biopsy™ IF Stainer is an automatic immunostaining system that allows simultaneous staining of a total of 12 slides. This system consists of a main X-Y axis module, a 1-ml pipette (ADP) module, a covering module, a consumable parts module, a vision parts module, and a consumable table. Smart Biopsy™ Cell Isolator is operated according to the manual using in-house software.

**Method for staining circulating tumor cells**

[Smart Biopsy™ IF Stainer method]

1. Cell-fixed slides and required antibodies (e.g., EpCAM, vimentin, PD-L1, CD18, and CD45 antibodies) are placed in buffer tubes inside the Smart Biopsy™ IF Stainer system, and then the system is operated according to the manual.
2. Washing, blocking, primary antibody/secondary antibody staining, and mounting processes are performed automatically for a total of 6 hours (based on 12 slides).
3. All staining processes by Smart Biopsy™ IF Stainer are performed according to the following manual method.

[Manual method]

1. The sample (slide) is treated with 50 ul of 0.2% Triton X-100 for 10 minutes, and then washed three times with PBS for 5 minutes.
2. Blocking is performed for 1 hour using 1% BSA to reduce non-specific binding.
3. Required antibodies (e.g., anti-CD45 for negative selection of immune cells, anti-CD18 for negative selection of lymphoid cells, and PD-L1 antibody for positive selection) are each diluted in 1% BSA, and the sample is treated with each antibody dilution for 1 hour (primary antibody treatment).
4. Two secondary antibodies ("goat Anti-mouse Alexa Fluor 647" and "goat Anti-rabbit Alexa Fluor 546") with two different wavelengths were diluted in 1% BSA, and then the sample is incubated with the secondary antibody dilution at room temperature for 1 hour (secondary antibody treatment).
5. A blocking process is performed for 1 hour to inhibit cross-reaction with an antibody (PD-L1) specific to lung cancer circulating tumor cells.
6. The sample is incubated with Anti-EpCAM Alexa Fluor 488-conjugated antibody, which is a marker for circulating tumor cells, at room temperature for 1 hour.
7. A washing process using PBS is performed 3 times for 5 minutes.
8. A DAPI staining process is performed, followed by a mounting process using a cover glass.

2-2. Selection of markers for CTC filtering CTC

In order to more clearly identify CTCs in blood, lymphoid cells were removed using CD45, which is known as a marker for lymphoid cells, and in order to remove false-positive cells (myeloid cells) other than CD45-positive cells, the expression levels of CD18 and CD16, known as specific markers, and the expression levels of CD18 and CD11b, in inflammatory cell lines, were analyzed by immunofluorescence staining (IF) .
The cell lines used were Jurkat (KCLB 40152) as human T lymphocyte cells, THP-1 (KCLB 40202) as human monocytic cells, and KG-1 (KCLB 10246) as human myeloblast cells. Information on an antibody for each antigen is shown in Table 5 below. 10 slides were stained with each of a sample of a CD18/CD16 combination and a sample of a CD18/CD11b combination, followed by analysis.

[Table 5]

| Antibody name | Manufacturer | Cat No. | Stock con. | Working rate |
|---|---|---|---|---|
| CD18(M) | NOVUS | MAB1730 | 1 mg/ml | 1:10 to 1:500 |
| CD18(M) | Thermo Fisher | 37-1300 | 0.5 mg/ml | 1:10 to 1:500 |
| CD18(R) | CST | 73663 | 60 μg/ml | 1:10 to 1:500 |
| CD16(M) | R&D | MAB2546-500 | 0.5 mg/ml | 1:10 to 1:500 |
| CD16(M) | NOVUS | NB500-378 | 1 mg/ml | 1:10 to 1:500 |

(continued)

| Antibody name | Manufacturer | Cat No. | Stock con. | Working rate |
|---|---|---|---|---|
| CD11b-FITC | Biolegend | 101206 | 0.5 mg/ml | 1:10 to 1:500 |

In this case, as secondary antibodies, Thermo Fisher's anti-rabbit IgG, Alexa Fluor 546 (A-11010/2 mg/mL) and antimouse IgG, Alexa Fluor 546 (A-11003/2 mg/mL) were used.

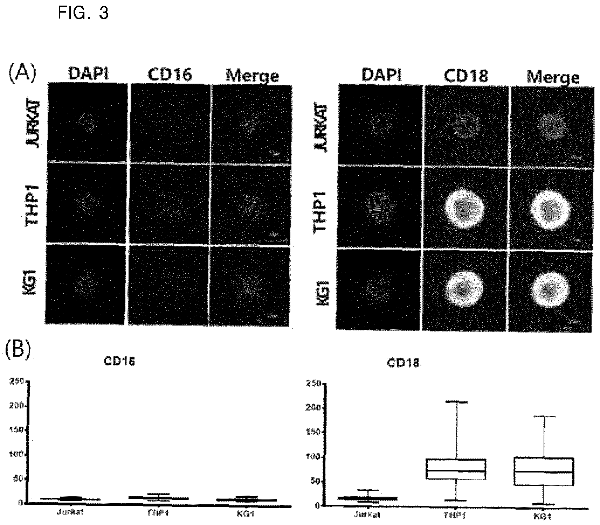

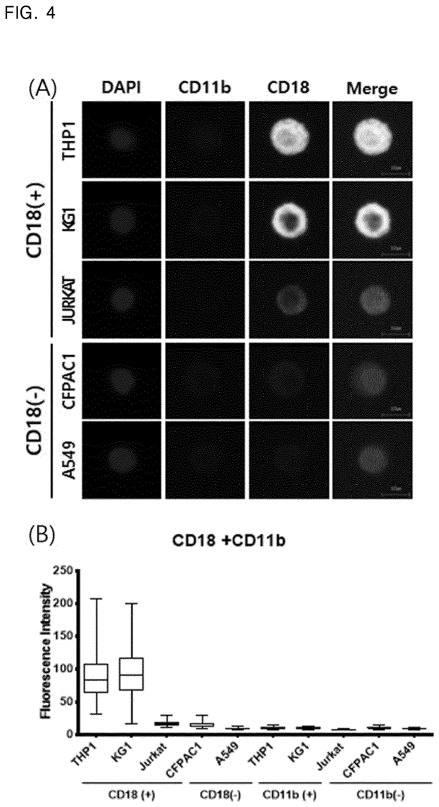

As a result, as shown in FIGS. 3 and 4, it was confirmed that CD18 was not only higher in expression than CD16 and CD11b but also important as a specific marker.

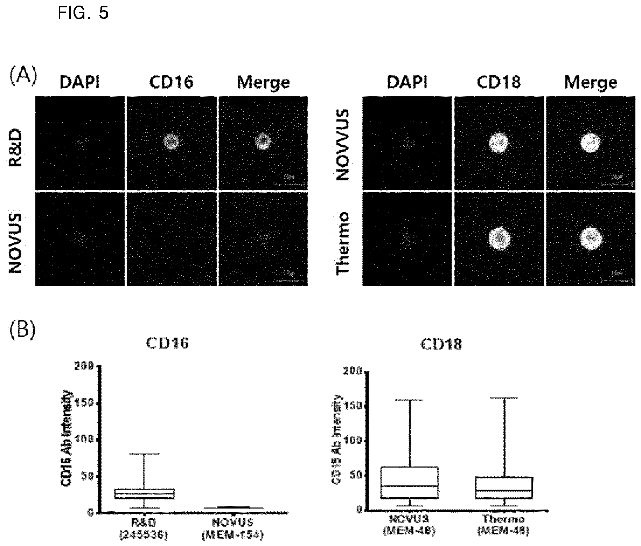

In addition, as a result of repeating the same experiment in PBMCs in patient blood, as shown in FIG. 5, it was confirmed that the expression level of CD18 was higher.

Accordingly, CD18 together with CD45 was selected as a marker for CTC filtering and used to eliminate false positive cells.

## Example 3. CTC staining based on selected markers

Cells (about 500 to 10,000 or more cells) isolated by the isolator were fixed onto a slide for staining by performing a cytospin process which is a cell centrifugation process.

The characteristics of the isolated CTCs were analyzed using CD45 and CD18 markers for negative selection and EpCAM/vimentin and PD-L1 markers for positive selection of CTCs.

As the antibodies, EpCAM/vimentin, PD-L1, CD18 and CD45 antibodies were used, and information on the antibodies is shown in Table 6 below. Finally, DAPI (4',6-diamidino-2-phenylindole) capable of staining cell nuclei was added and cell staining was performed using SmartBiopsy™ IF Stainer (CST030, CytoGen) according to the manufacturer's instructions.

[Table 6] Information on antibodies

| Antibody name | Manufacturer | Cat No. | Stock con. | Working rate |
|---|---|---|---|---|
| PD-L1 22C3 | Dako | M3653 | 150 mg/mL | 1:10 to 1:500 |
| CD18 (M) | Thermo Fisher | 37-1300 | 0.5 mg/mL | 1:10 to 1:500 |
| CD18 (R) | CST | 73663 | 60 μg/ml | 1:10 to 1:500 |
| EpCAM-488 | CST | 5198S | 100 μg/ml | 1:10 to 1:500 |
| Vimentin-488 | CST | 9854S | 200 μg/ml | 1:10 to 1:500 |
| CD45 | BD | 561864 | - | 50 μl |

## Example 4. Analysis of fluorescence images of CTCs and confirmation of accuracy of CTC isolation

Using SmartBiopsy™ Cell Image Analyzer (Cytogen), images of the slides loaded with fluorescently labeled CTCs were taken, and the numbers and fluorescence intensities of cells expressing EpCAM/vimentin, PD-L1 and CD18 were measured (FIGS. 1 and 2).

As a result of checking the expression of CTC markers and PD-L1 in the blood samples from the infected patients and lung cancer patients, it was confirmed that false positive cells expressing CTC markers and PD-L1 were present (FIGS. 1 and 2).

These false-positive cells can be regarded as myeloid cells expressing CD16/18. Thus, in the present invention, false-positive cells were excluded by using the CD16/18 marker as a single negative marker or a leukocyte-specific selective marker.

Accordingly, CD18(-) and PD-L1(+) cells were defined as pure PD-L1 expressing cells from which false positives were eliminated.

In addition, it was confirmed that CD18/CD45-negative CTCs were isolated from 21 out of the 29 lung cancer patients and 4 out of the 30 infected patients. As shown in Table 7 below, it was confirmed that the sensitivity of CTC isolation for lung cancer diagnosis was 72.4% and the specificity was 86.7%.

[Table 7] Accuracy of CTC isolation

|  | Total | Lung cancer group | Infection group |
|---|---|---|---|
| **CD18/CD45-negative CTCs** | 25 | 21 | 4 |
| **No CTC** | 34 | 8 | 26 |
| **Total** | 59 | 29 | 30 |

Sensitivity, 72.4%; Specificity, 86.7%

### Example 5. Analysis of correlation of PD-L1 expression level between CTCs and tissue sample

The results of analysis of PD-L1 expression in tissue samples are shown in Table 4 above, and patients with PD-L1-positive CTCs selected from the PBMCs from the 29 lung cancer patients according to the criteria of Example 4 were identified.

As a result, as shown in Table 8 below, it was confirmed that 14 out of the 29 patients were positive for PD-L1 expression in both the tissue samples and the blood samples, and 7 patients were negative for PD-L1 expression in both the tissue samples and the blood samples.

[Table 8] Correlation of PD-L1 expression between CTCs and tissue samples

|  |  | PD-L1-positive CTCs in peripheral blood | | Total |
|---|---|---|---|---|
|  |  | Negative | Positive |  |
| **PD-L1 expression in tumor tissue** | Negative | 7 | 5 | 12 |
|  | Positive | 3 | 14 | 17 |
| | Total | 10 | 19 | 29 |

Accuracy = 72.4%; Sensitivity = 82.4%; Specificity = 58.3%; Weighted Kappa = 0.417

Therefore, it was confirmed that the expression of PD-L1 in the tumor tissue could be predicted through the number of PD-L1-positive CTCs in the blood, with an accuracy of 72.4% and a sensitivity of 82.4%.

In addition, for the 21 patients having CTCs in the blood, the circulating tumor proportion score (CTPS) of PD-L1 was calculated by Equation 1 below, and the tumor proportion score (TPS) of PD-L1 was calculated by Equation 2 below.

[Equation 1]

$$CTPS = \frac{\text{Number of PD} - \text{L1 positive CTCs}}{\text{Total number of CTCs in blood}} * 100$$

[Equation 2]

$$TPS = \frac{\text{Number of PD} - \text{L1 positive tumor cells}}{\text{Total number of tumor cells in tissue sample}} * 100$$

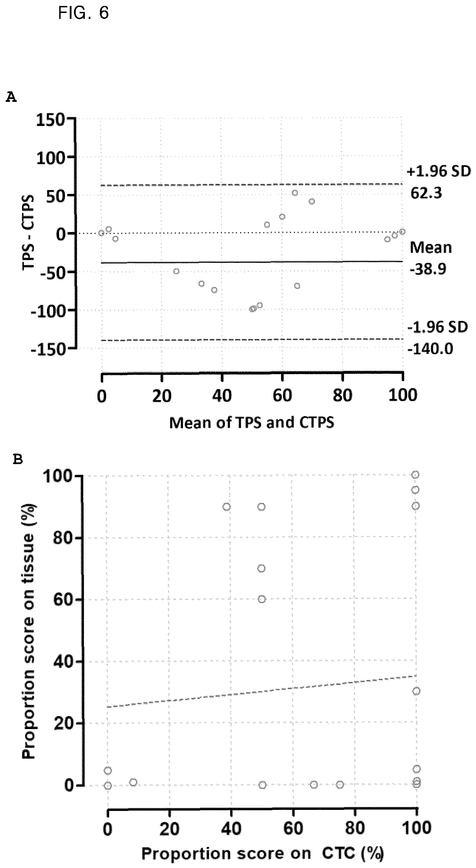

As a result, as shown in FIG. 6, it was confirmed that, as the TPS increased, the CTPS increased, and the CTPS was relatively low when the TPS was negative.

### Example 6. Prediction of tumor burden using CTPS

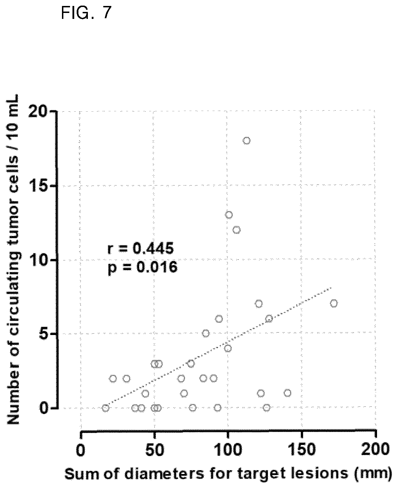

As a result of comparing the tumor sizes and CTPS values in the lung cancer patient tissue samples, as shown in FIG. 7, a Pearson correlation coefficient value of 0.445 was obtained and it was confirmed that there was a positive correlation between the two groups.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

INDUSTRIAL APPLICABILITY

In the method of treating cancer using circulating tumor cells according to the present invention, expression of immune checkpoint protein and expression of lymphoid cell- or myeloid cell-specific protein in a liquid biopsy are simultaneously checked, and then a patient is selected by eliminating false-positive circulating tumor cells. Thus, the method of the present invention has high accuracy and high commercial utility, and thus is useful for cancer diagnosis and treatment.

**Claims**

1.  A method for selecting a patient for application of a cancer immunotherapy drug, the method comprising steps of:

    (a) checking expression of immune checkpoint protein and lymphoid- or myeloid-specific protein in circulating tumor cells; and
    (b) classifying, as a patient to whom the cancer immunotherapy drug is applicable, the patient found to be positive for expression of the immune checkpoint protein and negative for expression of the lymphoid- or myeloid-specific protein.

2.  The method of claim 1, wherein the immune checkpoint protein is selected from the group consisting of PD-1, PD-L1, and CTLA-4.

3.  The method of claim 1, wherein the lymphoid- or myeloid-specific protein is selected from the group consisting of CD18, CD16, CD29, CD61, CD104, CD32, CD11b, and CD64.

4.  The method of claim 1, wherein the circulating tumor cells are isolated by performing the following steps:

    (i) obtaining blood from a cancer patient; and
    (ii) isolating circulating tumor cells from the blood using a biochip.

5.  The method of claim 1, wherein the expression of the immune checkpoint protein and the lymphoid- or myeloid-specific protein is checked by performing the following steps:

    (1) allowing circulating tumor cells to react with a fluorescent marker that binds specifically to the circulating tumor cells, a fluorescent marker that binds specifically to the immune checkpoint protein, and a fluorescent marker that binds specifically to the lymphoid- or myeloid-specific protein;
    (2) receiving optical images of the circulating tumor cells that reacted with the fluorescent marker, the immune checkpoint protein, and the lymphoid- or myeloid-specific protein in a plurality of wavelength ranges, respectively;
    (3) performing first filtering by measuring fluorescence intensities of the circulating tumor cells, the immune checkpoint protein, and the lymphoid- or myeloid-specific protein in the optical images in all or part of the plurality of wavelength ranges;
    (4) performing second filtering by measuring morphology of the circulating tumor cells in the optical images in all or part of the plurality of wavelength ranges; and
    (5) performing third filtering by measuring morphology of the circulating tumor cells in a combined image obtained by merging all or part of the optical images in the plurality of respective wavelength ranges.

6.  The method of claim 5, wherein the fluorescent marker that binds specifically to the circulating cancer cells is selected from the group consisting of an antibody specific for vimentin, an antibody specific for EpCAM, and an antibody for CK.

7.  The method of claim 5, wherein the fluorescent marker that binds specifically to the immune checkpoint protein is selected from the group consisting of an antibody specific to PD-1, an antibody specific to PD-L1, and an antibody specific to CTLA-4.

8.  The method of claim 5, wherein the fluorescent marker that binds specifically to the lymphoid- or myeloid-specific protein is selected from the group consisting of an antibody specific to CD18, an antibody specific to CD16, an antibody specific to CD29, an antibody specific to CD61, an antibody specific to CD104, an antibody specific to CD32, an antibody specific to CD11b, and an antibody specific to CD64.

9. The method of claim 1, wherein the immune checkpoint inhibitor is an antibody.

10. The method of claim 1, wherein the immune checkpoint inhibitor is a monoclonal antibody.

11. The method of claim 1, wherein the immune checkpoint inhibitor is a human antibody, a humanized antibody, or a chimeric antibody.

12. The method of claim 1, wherein the immune checkpoint inhibitor is any one or more selected from the group consisting of a PD-L1 antagonist, a PD-1 antagonist, and a CTLA-4 antagonist.

13. The method of claim 1, wherein the immune checkpoint inhibitor is any one or more selected from the group consisting of pembrolizumab (Keytruda), nivolumab (Opdivo), cemiplimab (Lybtayo), atezolizumab (Tecentriq), avelumab (Bacencio), durvalumab (Imfinzi), ipilimumab (Yervoy), and tremelimumab.

14. The method of claim 1, wherein the cancer is selected from the group consisting of lung cancer, kidney cancer, bladder cancer, breast cancer, colorectal cancer, ovarian cancer, pancreatic cancer, gastric carcinoma, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycosis fungoides, Merkel cell carcinoma, and hematologic malignancies.

15. The method of claim 14, wherein the cancer is lung cancer.

16. The method of claim 14, wherein the cancer is non-small-cell lung cancer.

17. A method for selecting cancer immunotherapy for a cancer patient, the method comprising steps of:

(a) checking expression of immune checkpoint protein and lymphoid- or myeloid-specific protein in circulating tumor cells; and
(b) selecting an immune checkpoint inhibitor as a therapeutic drug, when expression of the immune checkpoint protein is positive and expression of the lymphoid- or myeloid-specific protein is negative.

18. A method for treating cancer comprising steps of:

(a) checking expression of immune checkpoint protein and lymphoid- or myeloid-specific protein in circulating tumor cells; and
(b) administering an immune checkpoint inhibitor, when expression of the immune checkpoint protein is positive and expression of the lymphoid- or myeloid-specific protein is negative.

19. A method for determining susceptibility to an immune checkpoint inhibitor, the method comprising steps of:

(a) checking expression of immune checkpoint protein and lymphoid- or myeloid-specific protein in circulating tumor cells; and
(b) determining that the circulating tumor cells are susceptible to the immune checkpoint inhibitor, when expression of the immune checkpoint protein is positive and expression of the lymphoid- or myeloid-specific protein is negative.

FIG. 1

FIG. 2

(A)

(CTC marker : EpCAM/Vimentin, Myeloid marker: CD18)

(B)

(CTC marker : EpCAM/Vimentin, Myeloid marker: CD18)

FIG. 3

FIG. 4

FIG. 5

(A)

(B)

FIG. 6

FIG. 7

## INTERNATIONAL SEARCH REPORT

| International application No. |
| --- |
| **PCT/KR2022/005128** |

**A.    CLASSIFICATION OF SUBJECT MATTER**

**G01N 33/574**(2006.01)i; **G01N 33/50**(2006.01)i; **A61P 35/00**(2006.01)i; **A61K 39/00**(2006.01)i

According to International Patent Classification (IPC) or to both national classification and IPC

**B.    FIELDS SEARCHED**

Minimum documentation searched (classification system followed by classification symbols)

G01N 33/574(2006.01); A61K 39/395(2006.01); C07K 16/28(2006.01); C12Q 1/6886(2018.01); G01N 21/17(2006.01); G01N 33/15(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 면역 관문 억제제(immune checkpoint inhibitor), 혈중 순환 암세포(circulating tumor cell, CTC), 면역관문 단백질(immune checkpoint protein), 림프계 단백질(lymphatic protein), 골수계 단백질(bone marrow protein), 광학 이미지(optical image), 암(cancer)

**C.    DOCUMENTS CONSIDERED TO BE RELEVANT**

| Category* | Citation of document, with indication, where appropriate, of the relevant passages | Relevant to claim No. |
| --- | --- | --- |
| Y | KR 10-2018-0133465 A (CREATV MICROTECH, INC.) 14 December 2018 (2018-12-14) <br> See claim 4; and paragraphs [0024], [0025], [0053], [0054], [0089]-[0091] and [0131]. | 1-17,19 |
| Y | US 2019-0376146 A1 (DAIICHI SANKYO COMPANY, LIMITED) 12 December 2019 (2019-12-12) <br> See paragraph [0168]; and figure 4M. | 1-17,19 |
| Y | KR 10-1926117 B1 (CYTOGEN, INC.) 06 December 2018 (2018-12-06) <br> See claim 1; and paragraphs [0004] and [0006]-[0017]. | 5-8 |
| A | WO 2016-061064 A1 (EPIC SCIENCES, INC.) 21 April 2016 (2016-04-21) <br> See abstract; and claims 1-20. | 1-17,19 |
| A | WO 2019-143880 A1 (DANA-FARBER CANCER INSTITUTE, INC.) 25 July 2019 (2019-07-25) <br> See abstract; and claims 1-61. | 1-17,19 |

☐ Further documents are listed in the continuation of Box C.        ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **15 July 2022** | **15 July 2022** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2022/005128**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **18**
because they relate to subject matter not required to be searched by this Authority, namely:

Claim 18 pertains to a method for treatment of the human body by therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

## INTERNATIONAL SEARCH REPORT
### Information on patent family members

International application No.

**PCT/KR2022/005128**

| Patent document cited in search report | | | Publication date (day/month/year) | Patent family member(s) | | | Publication date (day/month/year) |
|---|---|---|---|---|---|---|---|
| KR | 10-2018-0133465 | A | 14 December 2018 | AU | 2017-250284 | A1 | 08 November 2018 |
| | | | | CA | 3020854 | A1 | 19 October 2017 |
| | | | | CN | 109311989 | A | 05 February 2019 |
| | | | | EP | 3443013 | A1 | 20 February 2019 |
| | | | | JP | 2019-514028 | A | 30 May 2019 |
| | | | | US | 2019-0128892 | A1 | 02 May 2019 |
| | | | | WO | 2017-181073 | A1 | 19 October 2017 |
| US | 2019-0376146 | A1 | 12 December 2019 | EP | 3546591 | A1 | 02 October 2019 |
| | | | | WO | 2018-097166 | A1 | 31 May 2018 |
| KR | 10-1926117 | B1 | 06 December 2018 | CN | 110945359 | A | 31 March 2020 |
| | | | | EP | 3671214 | A2 | 24 June 2020 |
| | | | | JP | 2020-529023 | A | 01 October 2020 |
| | | | | JP | 7040816 | B2 | 23 March 2022 |
| | | | | US | 2020-0225238 | A1 | 16 July 2020 |
| | | | | WO | 2019-035565 | A2 | 21 February 2019 |
| | | | | WO | 2019-035565 | A3 | 11 April 2019 |
| WO | 2016-061064 | A1 | 21 April 2016 | EP | 3207377 | A1 | 23 August 2017 |
| | | | | US | 2017-0242016 | A1 | 24 August 2017 |
| WO | 2019-143880 | A1 | 25 July 2019 | US | 2021-0072248 | A1 | 11 March 2021 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10502668 B

- KR 101254675

**Non-patent literature cited in the description**

- **HODI et al.** *N. Engl. J. Med.,* 2010, vol. 363, 711-23
- **HERBST RS et al.** *The New England Journal of Medicine.,* 2020, vol. 383 (14), 1328-39

- **RIJAVEC E et al.** *Cancers.,* 2020, vol. 12 (1), 17